# EUROPEAN PATENT SPECIFICATION

(11) **EP 2 491 997 B1**
(45) Date of publication and mention of the grant of the patent: **10.05.2017**
(21) Application number: 11155922.5
(22) Date of filing: 25.02.2011
(51) Int. Cl.: B01D 53/04, A61M 16/22, B01D 53/62, B01D 53/82

(54) **A housing for a solid or fluidal substance for the removal of an undesired respiratory gas component of a respiratory gas flow and an arrangement for ventilating lungs of a subject**
Gehäuse für eine feste oder fluidische Substanz zur Entfernung eines unerwünschten Atemgasbestandteils aus einem Atemgasstrom und Anordnung zur Beatmung der Lungen eines Patienten
Boîtier pour une substance solide ou fluide pour éliminer un composant indésirable du gaz respiratoire d'un flux de gaz respiratoire et agencement pour ventiler les poumons d'un sujet

(43) Date of publication of application: 29.08.2012
(73) Proprietor: CareFusion Corporation, San Diego, CA 92130 (US)
(72) Inventor: Heinonen, Erkki, 00750, Helsinki (FI)
(74) Representative: Farrington, Graham

(56) References cited:
- WO-A1-96/15027
- DE-C- 702 099
- GB-A- 2 420 507
- US-A- 3 752 654
- US-A- 4 717 549
- US-A1- 2004 079 367

## Description

### BACKGROUND OF THE INVENTION

This disclosure relates generally to a housing for solid, fluidal substance removing an undesired respiratory gas component of a respiratory gas flow and an arrangement for ventilating lungs of a subject.

Anesthesia machines are optimized for delivering anesthesia to a patient using volatile anesthetic agent liquids. In such systems, the anesthetic agent is vaporized and mixed into the breathing gas stream in a controlled manner to provide a gas mixture for anesthetizing the patient for a surgical operation. The most common volatile anesthetic agents are halogenated hydrocarbon chains, such as halothane, enflurane, isoflurane, sevoflurane and desflurane. Additionally, nitrous oxide (N₂O) can be counted in this group of volatile anesthetic agents, although the high vapor pressure of nitrous oxide causes nitrous oxide to vaporize spontaneously in the high pressure gas cylinder, where it can be directly mixed with oxygen. The anesthetizing potency of nitrous oxide alone is seldom enough to anesthetize a patient and therefore another volatile agent is used to support that.

Since the volatile anesthetic agents are expensive, they are effective greenhouse gases and further harmful to the atmospheric ozone layer, anesthesia machines have been developed to minimize the consumption of the gases. To keep patient's anesthetized, a defined brain partial pressure for the anesthetic agent is required. This partial pressure is maintained by keeping the anesthetic agent partial pressure in the lungs adequate. During a steady state, the lung and body partial pressures are equal, and no net exchange of the anesthetic agent occurs between the blood and the lungs. However, to provide oxygen and eliminate carbon dioxide, continuous lung ventilation is required.

Anesthesia machines are designed to provide oxygen to the patient and eliminate carbon dioxide (CO2), while preserving the anesthetic gases. To meet these goals a re-breathing circuit is used. In this patient exhaled gas is reintroduced for inhalation. Before re-inhalation carbon dioxide must be removed from the gas, which is done with a carbon dioxide absorber. Before inhalation, the gas is supplied with additional oxygen and anesthetic agents based upon the patient demand. In this arrangement, the additional gas flow added to the re-breathing circuit can be less than 0.5 L/min although the patient ventilation may be 5-10 L/min. Such ventilation of the lung is carried out using a ventilator pushing inhalation gas with overpressure to patient lungs and then allowing that to flow out passively from the pressurized lungs to the breathing circuit.

Ventilation carries the breathing circuit oxygen to lungs for uptake to be burned in body metabolism. The outcome of this is CO2 that diffuses to lungs and becomes carried out with exhalation gas. Before re-inhalation the gas is guided through absorber for CO2 removal. Effective CO2 removal requires close contact with the breathing gas and the removing substance. To provide large contact area, the removing substance is therefore a surface of porous structure of granules that fill the cartridge. The form of this granular structure guided by the flow resistance, the limitation of which is one key design goals of the breathing circuit. In absorber optimized for minimal resistance the gas flow path through the stacked granules is short and the flow distributes to wide area. In such structure the gas flows slowly because of large surface area providing time for reaction between the gas and absorbent granules.

However, such optimal wide and short cartridge design involves a problem. Because the removing material is in granules, those may move in relation to each other. Packaging of the granules into the cartridge occurs in a factory, and thereafter the cartridge is transported to customer site. During this transport the granules are experiencing shaking. This compresses the granules increasing granule packaging grade and reduces the volume of the granule bed in the cartridge. Therefore when taken into use the cartridge may have some empty space on its top. Because of this reality, the gas flows through the absorber vertically, since in horizontal flow the gas (favoring the route of the least resistance to flow) would flow through this empty space without communication with the absorbent and thus allowing the CO2 flow through the absorber.

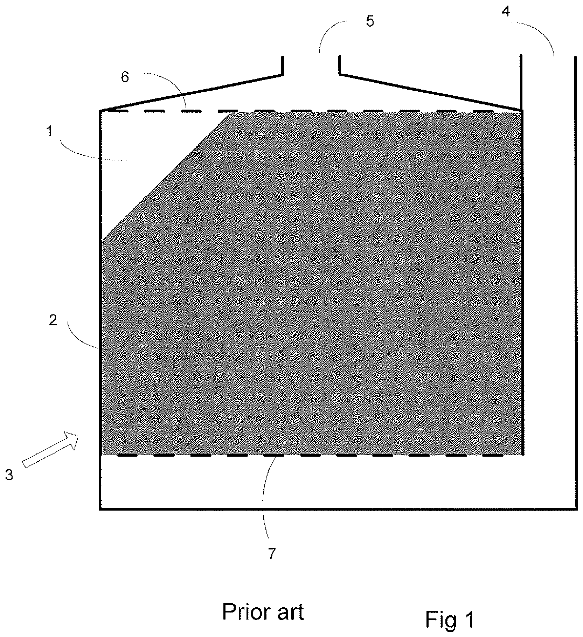

In the vertical gas flow this horizontal empty space is not for any harm since that does not disturb the internal flow resistance distribution within the cartridge. However, if the top surface of the granules is slanted as shown in Figure 1, flow density at the low granule level, where the empty space 1 exists, increases over the areas of high level of granules 2. On CO2 absorber language this is known as a flow channeling. A typical cartridge 3 comprises gas input 4, a gas output 5 and mesh plates 6 and 7 for preventing granules therebetween to escape from the cartridge. A demand of CO2 removal is proportional to the flow rate and the absorbent is used locally more at the volumes of high gas flow. In these areas also the absorbent volume is smaller. These result fast absorbent wear-out on these high flow volumes. When all absorbent has been consumed the CO2 penetrates through the cartridge using this flow path wherefrom the capacity has ended increasing the inspired patient gas CO2 concentration. This signs for cartridge wear out although still unused material may exists on the reduced flow volumes. Therefore slanting reduces usable cartridge CO2 removal capacity. The surface level of the granules may get slanted if inclined during unpacking and installing the cartridge having empty space after transport.

German Patent publication 702099 describes a carbon dioxide scrubber cartridge in which a plurality of horizontal separators are used to try to avoid short circuits in gals flow through the scrubber.

US Patent 4717549 describes an apparatus for oxygen generation in one embodiment of which vertical limiters are shown extending from a horizontal separator.

### BRIEF DESCRIPTION OF THE INVENTION

The above-mentioned shortcomings, disadvantages and problems are addressed herein which will be understood by reading and understanding the following specification. Accordingly, the present invention provides a housing including solid fluidal granules of substance for removing an undesired respiratory gas component of a respiratory gas flow as specified in the claims.

In an embodiment, the invention relates to a housing according to claim 1.

In another embodiment, the invention relates to an arrangement for ventilating lungs of a subject according to claim 11.

### BRIEF DESCRIPTION OF THE DRAWINGS

Figure 1 is a schematic view of prior art cartridge when returned to normal position after tilting;
Figure 2 illustrates an operational diagram of an arrangement for ventilating lungs of a subject:
Figure 3 is a schematic view of a housing for a solid fluidal substance removing an undesired respiratory gas component of a respiratory gas flow in accordance with an embodiment;
Figure 4 shows a behavior of the substance when the housing of Figure 3 has been returned to the formal position after tilting;
Figure 5 is a cross section of the housing of Figure 3 taken along lines A - A in accordance with an embodiment;
Figure 6 is a cross section of the housing of Figure 3 along lines A - A according to another embodiment;
Figure 7 is a schematic view of a housing for a solid fluidal substance removing an undesired respiratory gas component of a respiratory gas flow in accordance with another embodiment; and
Figure 8 is a cross section of the housing of Figure 7 taken along lines B - B.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

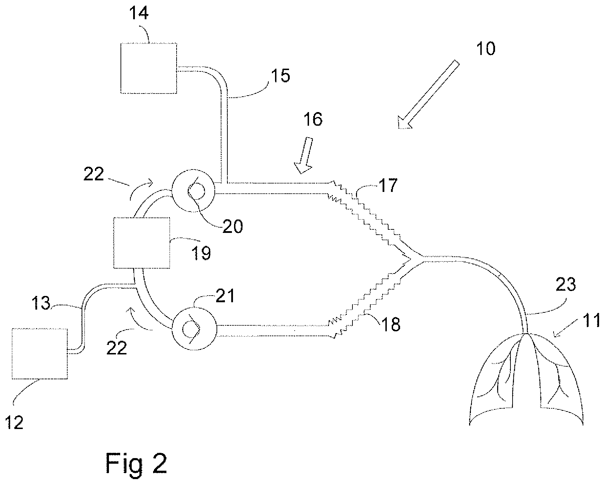

In Fig. 2 an arrangement 10 for ventilating lungs 11 of a subject is disclosed. The arrangement comprises a ventilator 12 supplying in this specific embodiment along a tube 13 breathing gas to the lungs for an inspiration and receiving breathing gas for an expiration. The ventilator may be whichever well-known type e.g. drive gas based pneumatic flow-valve or mechanical piston driven. Also the arrangement comprises a gas mixer 14 supplying a fresh gas in this specific embodiment along a fresh gas tube 15 for the subject breathing, a breathing circuit 16 connecting lungs of the subject, the ventilator 12 and the gas mixer 14. The gas mixer may comprise an anesthetic agent supply (not shown in the figure) such as an anesthetic agent vaporizer providing anesthetic agent for the subject breathing.

The breathing circuit 16, which may be a re-breathing circuit, comprises an inspiration limb 17 providing an inspiration gas including the fresh gas for the subject breathing and an expiration limb 18 discharging an expiration gas. The ventilator is controlling the breathing circuit pressure through the tube 13. Also the breathing circuit comprises a housing 19 for a solid fluidal substance such as granules for removing an undesired respiratory gas component of a respiratory gas flow. Typical solid, fluidal substance used in anesthesia is a carbon dioxide absorbing material, which may be soda-lime, a mixture of calcium hydroxide, sodium hydroxide, potassium hydroxide and water or any other substance that can extract CO2 from gas mixture e.g. molecular sieve or amines. The material may chemically react with carbon dioxide or otherwise remove it from the breathing gas. The housing 19 may be detachable from the breathing circuit. Typically the breathing circuit 16 also comprises directional valves 20 and 21 guiding the gas flow in the circuit on direction indicated by arrows 22. For inhalation the ventilator 12 increases the breathing circuit pressure by adding the gas flow from tube 13. Directional valves 20 and 21 guide the gas flow through the housing 19, including the substance removing in this embodiment carbon dioxide from the breathing gas, to the inspiration limb 17 and further along a subject limb 23 to the subject's lungs 11. For expiration the ventilator 12 releases gases from the breathing circuit through the tube 13. For this purpose the ventilator 12 may e.g. operate an expiration valve (not shown in Figure). This will allow the gas flow from distended subject's lungs through the subject limb 23 to the expiration limb 18 and further through the directional valve 21 to the tube 13. The directional valve 20 prevents the gas flow from the subject's lungs to enter the inspiration limb 17 hereby maintaining the inspiration limb free from CO2. Instead, the exhaled gas is rich of CO2 that needs to be removed before being re-circulated for the inspiration, which is done in the housing 19 including the substance removing carbon dioxide.

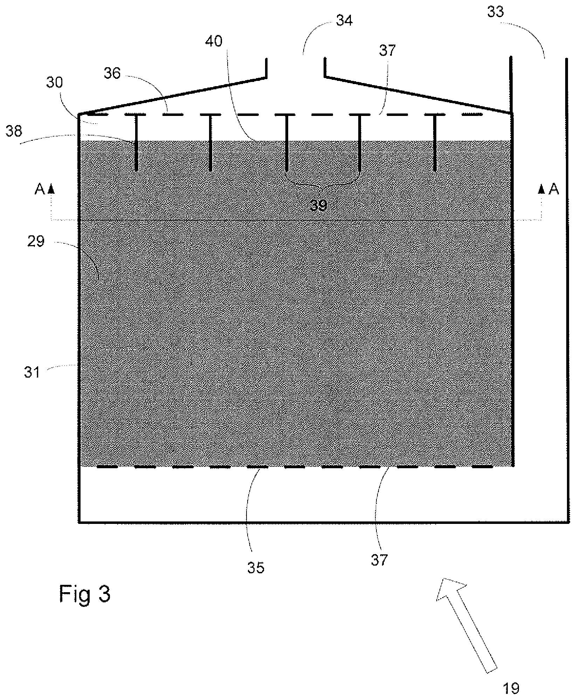

Fig. 3 presents a schematic drawing of the housing 19 comprising a space 30 receiving the solid fluidal substance 29 used in this embodiment to absorb carbon dioxide of the respiratory gas and a wall 31 surrounding a part of the space 30. Through the gas inlet 33 the respiratory gas is flowing to the housing and guided through the space filled with the substance 29 to the gas outlet 34 for leaving the housing and the space 30. Depending on the application the gas may flow on either direction through the space 30 of the housing 19. The housing further comprises a first separator 35 and a second separator 36, which may be operatively connected or connectable to the wall. The first separator 35 such as a mesh plate allows the gas flow but prevents the substance escaping from the space through the first separator and the gas inlet 33. The second separator 36 such as a mesh plate allows the gas flow but prevents the substance escaping from the space through the second separator and the gas outlet 34. It should be noted, because the gas may flow on either direction through the space 30 of the housing 19, one of the first separator and the second separator allows the respiratory gas flow to the space and the remaining one of the first separator and the second separator lets the respiratory gas flow from the space. The substance 29 is thus hold on within the housing with the first and second separator allowing the gas flow through openings 37 holding the access of the absorbent granule of the substance 29 through these openings. These first and second separators may be separate layers within the housing 19 as indicated on the Fig. 3 or they may form the top and bottom ends of the housing depending on the breathing system connection interface. Further the gas inlet 33 and the gas outlet 34 can be separate and, if desired, distant from the first separator and the second separator or the first separator can also be one of the gas inlet and the gas outlet and the second separator can correspondingly be one of the remaining one of the gas inlet and the gas outlet.

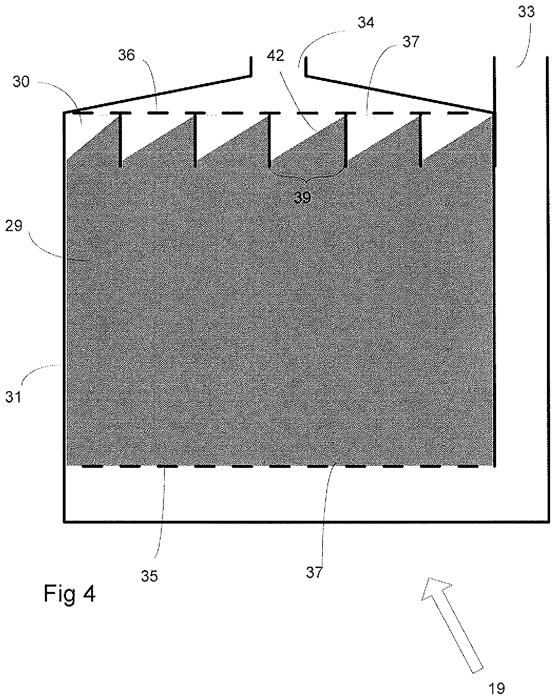

The housing further comprises a limiter 38 between the first separator 35 and the second separator 36 to limit a movement of the solid fluidal substance inside the space 30. In Fig. 3 from the top second separator 36 extends the limiter 38 towards the space 30 into the substance. Also the limiter can be extended from the first separator 35 towards the space 30 and into the substance, if it is only useful. This is especially in case it is possible to turn the housing upside down. The limiter 38 is closer to one of the first separator and second separator than midway between the first separator and the second separator. Also both the limiter 38 and one of the first separator 35 and the second separator 36 can constitute an integral structure. The limiter can also extend from both the first and second separator towards the space 30 making sure that the housing can be assembled on both ways. Thus the limiter can be an extension of one of the first separator and the second separator and can be long enough to reach the substance 29 even after packing of the granules of the substance during transport. The limiter may extend from one of the first separator and the second separator towards the space at least 2 or more specifically 2,5 times a distance which is estimated to form between one of the first separator and the second separator and an upper level 40 of the substance when compressed at the time taken into use. Advantageously even at the lowest level the substance extends vertically half-way up the dimension of the limiter. When so, sloping of the substance after resuming the housing tilting remains distributed within the individual compartments 39 conformed by the limiter as indicated in Fig 4. Thus the compartments 39 formed by the limiter are able to limit the movement of the substance to various directions following one of the first separator and the second separator through the space. The limiter can be also separate from one of the first separator and the second separator but still that may locate close enough to limit the granules of the substance from flowing through this separation when tilting.

The vertical dimension of the limiter 38 is advantageously limited since all walls may enhance the channeling effect. Therefore uniform volume of the granules of the substance 29 either before or after the penetrating respiratory gas enters the limiter area is beneficial to guarantee effective CO2 removal despite of the potential local channeling caused by the limiter 38.

The maximum angle the granules of the substance can settle determines the optimal dimensioning of the limiter including a horizontal distance of the adjacent extensions and a vertical height or depth of the limiter. Depending on the granules of the substance, maximum sloping angle 42 can be up to 45 degrees as shown in Fig. 4. Therefore the vertical height and horizontal distance of the limiter is advantageously equal. If the potential sloping angle is smaller then the horizontal distance may be longer than the vertical height. Horizontal distance smaller than vertical height does not provide any additional benefit but may instead prevent filling of the limiter space. For practical solutions of different granules the horizontal distance to vertical height may vary from 2:1 to 1:1. Typically the packaging of the substance leaves less than 10% vertical empty space below the top separator, which may be one of the first separator and the second separator depending on which one is upwards. If the height of the volume of the substance is 10 cm, the empty space may thus be 1 cm and the required vertical dimension of the limiter is then at least 2 cm or even 2,5 cm, which is also advantageous horizontal distance of the limiter compartment 39.

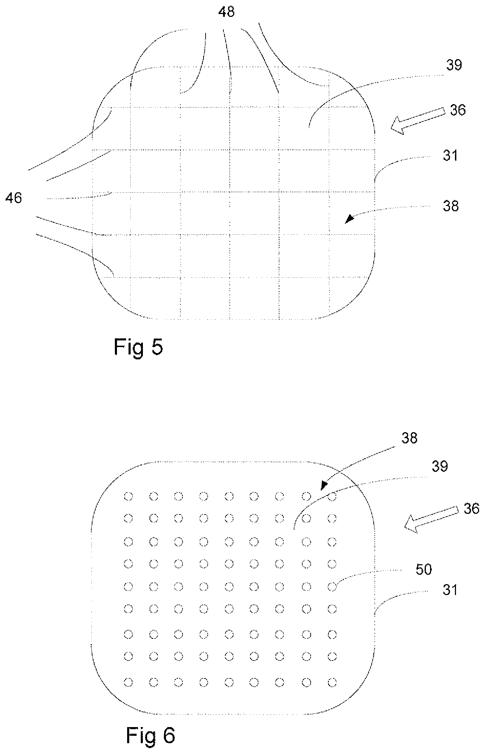

Fig. 5, which is a cross-section of the housing of Fig.3 along lines A - A, shows one embodiment of the second separator 36 having limiter 38 constituting an integral structure. The limiter comprises a first set of adjacent extensions 46 forming in this specific case a continuous structure following the second separator 36 through the space 30 when the housing is in the upright position and the second separator is substantially in the horizontal position and in which case the respiratory gas flow through the space happens in substantial vertical direction. The limiter shown in Fig. 5 also comprises a second set of adjacent extensions 48 which may also form in this specific embodiment a continuous structure following the second separator 36 through the space 30 when the housing is in the upright position and the second separator is substantially in the horizontal position and in which case the respiratory gas flow through the space happens in substantial vertical direction. The first set of adjacent extensions and the second set of adjacent extensions are crossing forming intersecting walls that form compartments 39. The first set of adjacent extensions may form an angle of 60-90 degrees, more specifically 70-90 degrees, or even more specifically 80-90 degrees with the second set of adjacent extensions. Adjacent extensions of the first set can be substantially parallel with each other. Also adjacent extensions of the second set can be substantially parallel with each other. As well the embodiment of Fig. 5 could describe the first separator 35 with the limiter 38 having same construction.

Fig. 6 shows a cross-section of the housing of Fig. 3 along lines A - A according to another embodiment of the second separator 36 having the limiter 38. The limiter comprises a set of pins 50, leaving therebetween compartments 39, following the second separator 36 through the space 30 when the housing is in the upright position and the second separator is substantially in the horizontal position and in which case the respiratory gas flow through the space happens in substantial vertical direction. The dimensional calculation above applies only for the structure of Fig 5. The pins 50 must be denser than adjacent extensions having continuous structure as shown in Fig. 5, because the granules of the substance may flow between the pins. Thus the distance between different pins can be from 5 to 15mm, more specifically from 7 to 13 mm or even more specifically 10 mm. Also other type of compartments 39, e.g. a honeycomb would meet the requirement of Fig. 5 and 6. An advantage of the structure is that it limits the solid fluidal substance movement independently of tilting direction and thus the compartmental division may occur on both horizontal directions. As well the embodiment of Fig. 6 could describe the first separator 35 with the limiter 38 having same construction.

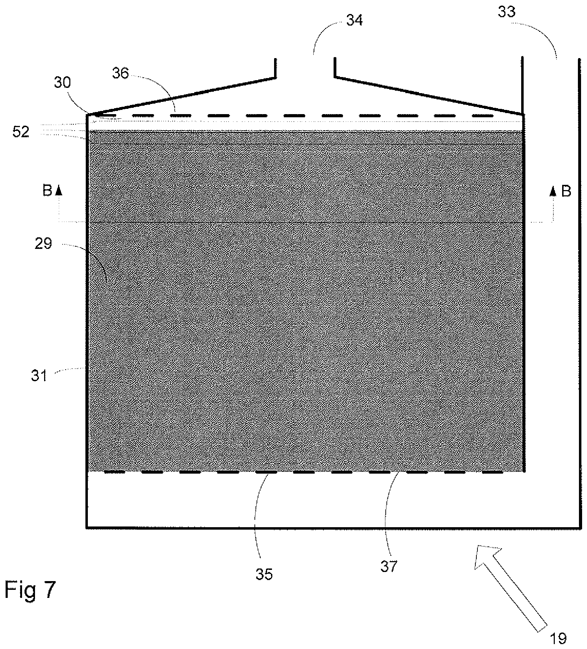

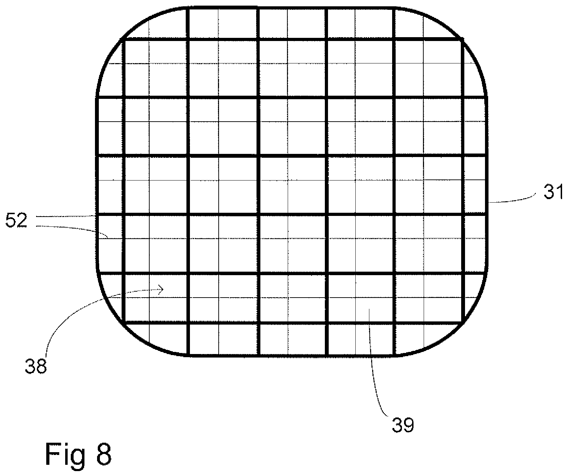

Fig. 7 shows another embodiment of the housing close to the embodiment of Fig. 3. The limiter 38 comprises a set of nets 52 one on the other. The nets are in horizontal position being substantially parallel with the second separator 36. Compartments 39 are formed between the nets. Distance between the nets and the closest net to the second separator 36 can be smaller than 10 mm. Distance between parallel net threads can be smaller than 20 mm. The nets may be interleaved to provide better grid against movement of the fluidic granules when tilting. As well the embodiment of Fig. 7 and 8 could describe the first separator 35 having the limiter 38 with same properties.

As hereinabove explained the embodiment discloses the housing and arrangement where slanting of solid fluidal substance, especially CO2 removing substance, is limited in an incompletely filled housing. For this purpose the top of the housing has the limiter extending into the substance, which may comprise absorbent granules. The limiter may extend to the top surface of the substance accommodating any empty space within the housing because of incomplete filling or stacking of the substance during transport. When tilting the housing the limiter limits flow of granules of the substance to one side of the housing and leaving empty space to the other when turning back to usage position. Instead, the limiter distributes this one empty space to a number of small compartments within the volume spaces formed by the limiter. As a result of this the height differences of the surface low's and high's is reduced when the empty space of the housing remains more evenly distributed at the top of the housing as shown in Fig. 4. This maintains even flow distribution throughout the space filled with the substance reducing the channeling and thus improving the usage of the substance, especially CO2 removal capacity.

The written description uses examples to disclose the invention, including the best mode, and also to enable any person skilled in the art to make and use the invention. The patentable scope of the invention is defined by the claims.

## Claims

1. A housing (19) for solid, fluidal substance removing an undesired respiratory gas component of a vertical respiratory gas flow comprising:
a space (30) for receiving said substance;
a vertical wall (31) surrounding part of said space;
a first horizontal separator (35) surrounding a top part of said space allowing the gas flow but preventing said substance escaping from said space through said first separator; and
a second horizontal separator (36) surrounding a bottom part of said space allowing gas flow but preventing said substance escaping from said space through said second separator;
wherein one of said first separator and said second separator is adapted to allow the respiratory gas flow to said space and the remaining one of said first separator and said second separator is adapted to let the respiratory gas flow from said space, said housing further comprising
a limiter (38) in form of a first set of adjacent extensions extending from said first horizontal separator into the substance,
the horizontal distance between adjacent extensions and the vertical height of the extensions being equal

2. The housing according to claim 1, **characterized in that** said limiter (38) and said first separator (35) is adapted to constitute an integral structure.

3. The housing according to claim 1, **characterized in that** said limiter is adapted to form compartments (39) to limit the movement of the substance to various directions following one of said first separator and said second separator through said space.

4. The housing according to claim 1, **characterized in that** said limiter is adapted to form a continuous structure following one of said first separator and said second separator through said space.

5. The housing according to claim 1, **characterized in that** said first set of_adjacent extensions are substantially parallel.

6. The housing according to claim 7, **characterized in that** said limiter also comprises a second set of adjacent extensions (48) crossing with said first set of extensions.

7. The housing according to claim 6, **characterized in that** said first set of extensions are adapted to form an angle of 60-90 degrees, more specifically 70-90 degrees, or even more specifically 80-90 degrees, with said second set of adjacent_extensions.

8. The housing according to claim 1, **characterized in that** said limiter comprises a set of pins (50) arranged side by side.

9. The housing according to claim 1, **characterized in that** said limiter comprises a set of nets (52) one on the other.

10. The housing according to claim 1, **characterized in that** said substance is a chemical compound removing carbon dioxide.

11. An arrangement for ventilating lungs of a subject comprising:
a ventilator (12) for supplying a breathing gas for an inspiration and for receiving a breathing gas for an expiration; and
a gas mixer (14) for supplying a fresh gas for the subject breathing;
a breathing circuit (16) for connecting lungs (11) of the subject, said ventilator and said gas mixer, said breathing circuit comprising an inspiration limb (17) providing an inspiration gas including the fresh gas for the subject breathing; an expiration limb (18) to discharge an expiration gas; and a housing (19) according to any of the preceding claims

## Patentansprüche

1. Gehäuse (19) für eine feste, fluidale Substanz, die eine unerwünschte Atemgaskomponente eines vertikalen Atemgasstroms entfernt, wobei das Gehäuse folgendes umfasst:
einen Raum (30) zur Aufnahme der genannten Substanz;
eine vertikale Wand (31), die einen Teil des genannten Raums umgibt;
einen ersten horizontalen Separator (35), der einen oberen Teil des genannten Raums umgibt, der den Gasfluss zulässt, es jedoch verhindert, dass die genannte Substanz durch den genannten ersten Separator aus dem genannten Raum austritt; und
einen zweiten horizontalen Separator (36), der einen unteren Teil des genannten Raums umgibt, der den Gasfluss zulässt, es jedoch verhindert, dass die genannte Substanz durch den genannten zweiten Separator aus dem genannten Raum austritt;
wobei ein Separator des genannten ersten Separators und des genannten zweiten Separators es ermöglichen kann, dass das Atemgas in den genannten Raum fließt, und wobei der verbleibende Separator des genannten ersten und zweiten Separators das Atemgas aus dem genannten Raum fließen lassen kann, wobei das genannte Gehäuse ferner folgendes umfasst:
einen Begrenzer (38) in Form einer ersten Anordnung benachbarter Extensionen, die sich von dem genannten ersten horizontalen Separator in die Substanz erstrecken;
wobei der horizontale Abstand zwischen benachbarten Extensionen und die vertikale Höhe der Extensionen identisch sind.

2. Gehäuse nach Anspruch 1, **dadurch gekennzeichnet, dass** der genannte Begrenzer (38) und der genannte erste Separator (35) eine integrale Struktur bilden können.

3. Gehäuse nach Anspruch 1, **dadurch gekennzeichnet, dass** der genannte Begrenzer Kammern (39) bilden kann, um die Bewegung der Substanz in verschiedene Richtungen zu begrenzen, einem Separator des genannten ersten Separators und des genannten zweiten Separators durch den genannten Raum folgend.

4. Gehäuse nach Anspruch 1, **dadurch gekennzeichnet, dass** der genannte Begrenzer eine ununterbrochene Struktur bilden kann, die einem Separator des genannten ersten Separators und des genannten zweiten Separators durch den genannten Raum folgt.

5. Gehäuse nach Anspruch 1, **dadurch gekennzeichnet, dass** die genannte erste Anordnung benachbarter Extensionen im Wesentlichen parallel ist.

6. Gehäuse nach Anspruch 1, **dadurch gekennzeichnet, dass** der genannte Begrenzer ferner eine zweite Anordnung benachbarter Extensionen (48) umfasst, welch die genannte erste Anordnung von Extensionen kreuzen.

7. Gehäuse nach Anspruch 6, **dadurch gekennzeichnet, dass** die genannte erste Anordnung von Extensionen einen Winkel von 60 bis 90 Grad, insbesondere von 70 bis 90 Grad oder noch präziser von 80 bis 90 Grad mit der genannten zweiten Anordnung benachbarter Extensionen bilden kann.

8. Gehäuse nach Anspruch 1, **dadurch gekennzeichnet, dass** der genannte Begrenzer eine erste Anordnung von Stiften (50) umfasst, die Seite an Seite angeordnet sind.

9. Gehäuse nach Anspruch 1, **dadurch gekennzeichnet, dass** der genannte Begrenzer eine Anordnung von übereinander angeordneten Netzen (52) umfasst.

10. Gehäuse nach Anspruch 1, **dadurch gekennzeichnet, dass** die genannte Substanz eine chemische Verbindung ist, die Kohlendioxid entfernt.

11. Anordnung zur Ventilation der Lunge eines Subjekts, wobei die Anordnung folgendes umfasst:
einen Ventilator (12) für die Zufuhr eines Atemgases für eine Inspiration und für den Empfang eines Atemgases für eine Expiration; und
einen Gasmischer (14) für die Zufuhr eines frischen Gases für das atmende Subjekt;
einen Atemkreislauf (16) zur Verbindung der Lunge (11) des Subjekts, des genannten Ventilators und des genannten Gasmischers, wobei der genannte Atemkreislauf ein Inspirationsglied (17) umfasst, das ein Inspirationsgas bereitstellt, welches das frische Gas für das atmende Subjekt aufweist; ein Expirationsglied (18) zur Ausgabe eines Expirationsgases; und ein Gehäuse (19) nach einem der vorstehenden Ansprüche.

## Revendications

1. Boîtier (19) pour une substance solide ou fluide éliminant un composant indésirable du gaz respiratoire d'un flux de gaz respiratoire vertical comprenant :
un espace (30) pour recevoir ladite substance ;
une paroi verticale (31) entourant une partie dudit espace ;
un premier séparateur horizontal (35) entourant une partie supérieure dudit espace autorisant le flux de gaz mais empêchant ladite substance de s'échapper dudit espace à travers ledit premier séparateur ; et
un second séparateur horizontal (36) entourant une partie inférieure dudit espace autorisant le flux de gaz mais empêchant ladite substance de s'échapper dudit espace à travers ledit second séparateur ;
l'un dudit premier séparateur et dudit second séparateur étant conçu pour autoriser le flux de gaz respiratoire vers ledit espace et ledit premier séparateur et ledit second séparateur restant étant conçu pour laisser le gaz respiratoire s'écouler dudit espace, ledit boîtier comprenant en outre
un limiteur (38) sous la forme d'un premier ensemble d'extensions adjacentes s'étendant dudit premier séparateur horizontal dans la substance,
la distance horizontale entre les extensions adjacentes et la hauteur verticales des extensions étant égale.

2. Boîtier selon la revendication 1, **caractérisé en ce que** ledit limiteur (38) et ledit premier séparateur (35) sont conçus pour constituer une structure intégrée.

3. Boîtier selon la revendication 1, **caractérisé en ce que** ledit limiteur est conçu pour former des compartiments (39) pour limiter le mouvement de la substance dans différentes directions en suivant ledit premier séparateur ou ledit second séparateur à travers ledit espace.

4. Boîtier selon la revendication 1, **caractérisé en ce que** ledit limiteur est conçu pour former une structure continue en suivant ledit premier séparateur ou ledit second séparateur à travers ledit espace.

5. Boîtier selon la revendication 1, **caractérisé en ce que** ledit premier ensemble d'extensions adjacentes est sensiblement parallèle.

6. Boîtier selon la revendication 1, **caractérisé en ce que** ledit limiteur comprend également un second ensemble d'extensions adjacentes (48) croisant ledit premier ensemble d'extensions.

7. Boîtier selon la revendication 6, **caractérisé en ce que** ledit premier ensemble d'extensions est conçu pour former un angle de 60 à 90 degrés, plus précisément de 70 à 90 degrés, ou même plus précisément de 80 à 90 degrés, avec ledit second ensemble d'extensions adjacentes.

8. Boîtier selon la revendication 1, **caractérisé en ce que** ledit limiteur comprend un ensemble de broches (50) disposées côte à côte.

9. Boîtier selon la revendication 1, **caractérisé en ce que** ledit limiteur comprend un ensemble de filets (52) l'un sur l'autre.

10. Boîtier selon la revendication 1, **caractérisé en ce que** ladite substance est un composé chimique éliminant le dioxyde de carbone.

11. Agencement pour la ventilation des poumons d'un sujet comprenant :
un ventilateur (12) pour administrer un gaz respiratoire pour une inspiration et pour recevoir un gaz respiratoire pour une expiration ; et
un mélangeur de gaz (14) pour administrer un gaz frais pour la respiration du sujet ;
un circuit de respiration (16) pour relier les poumons (11) d'un sujet, ledit ventilateur et ledit mélangeur de gaz, ledit circuit respiratoire comprenant une branche inspiratoire (17) fournissant un gaz d'inspiration comprenant le gaz frais pour la respiration du sujet ; une branche expiratoire (18) pour évacuer un gaz d'expiration ; et un boîtier (19) selon l'une quelconque des revendications précédentes.
